# FASCICULE DE BREVET EUROPEEN

(11) **EP 1 086 363 B1**
(45) Date de publication et mention de la délivrance du brevet: **09.01.2002**
(21) Numéro de dépôt: 99917005.3
(22) Date de dépôt: 06.05.1999
(51) Int. Cl.: G01N 11/16, G01N 33/49

(54) **APPAREIL D'ANALYSE AUTOMATIQUE UTILISABLE POUR LA DETERMINATION DU TEMPS DE COAGULATION DU SANG**
AUTOMATISCHE ANALYSEVORRICHTUNG, ZUR FESTSTELLUNG DER BLUTKOAGULATIONSZEIT ANWENDBAR
AUTOMATIC ANALYSIS APPARATUS USED FOR TIMING BLOOD COAGULATION

(30) Priorité: 10.06.1998 FR 9807484
(43) Date de publication de la demande: 28.03.2001
(73) Titulaire: Junior Instruments, 92230 Gennevilliers (FR)
(72) Inventeur: ROUSSEAU, Alain, F-75004 Paris (FR)
(74) Mandataire: de Saint-Palais, Arnaud Marie
(86) Numéro de dépôt international: FR9901082
(87) Numéro de publication internationale: WO9964839

(56) Documents cités:
- EP-A- 0 325 874
- EP-A- 0 525 273
- DE-A- 3 540 661
- DE-A- 3 738 375
- US-A- 4 612 801
- US-A- 5 394 739

## Description

La présente invention concerne un appareil d'analyse automatique utilisable notamment à la détermination des temps de modification de l'état physique d'un milieu fluide.

Elle s'applique tout particulièrement, mais non exclusivement, à la détermination du temps de coagulation du sang conformément à un processus selon lequel l'échantillon de sang est disposé dans le fond d'une cuvette contenant une bille ferromagnétique entraînée dans un mouvement périodique sous l'effet d'un champ magnétique extérieur. Les modifications des mouvements de la bille ferromagnétique (par exemple les variations d'amplitude et/ou de fréquence) qui sont représentatives des changements de l'état physique du sang, sont alors détectées à l'aide de moyens appropriés (optoélectronique - inductif, capacitif, densitométrique).

Le principe d'une telle détection se trouve exposé dans le brevet européen No EP 88403279.8 déposé au nom de la Société SERBIO S.A.

Dans un appareil d'analyse à cadence élevée de ce genre (de l'ordre de 360 tests/heure), chaque cuvette, à usage unique, présente une forme parallélépipédique de faibles dimensions, dont le fond incurvé constitue le chemin de roulement de la bille, tandis que la face opposée au fond présente une ouverture. Ces godets sont disposés côte à côte et fixés de façon détachable sur une bande de support souple qui obture temporairement leurs ouvertures. La bande équipée de godets peut être enroulée sur une bobine pouvant s'engager sur un moyeu prévu dans un compartiment de stockage et de distribution d'un automate.

Dans ce type de machines automatiques dont le taux de défaillance toutes causes confondues est de l'ordre de 3 interventions/an, les godets sont extraits de la bande pour être ensuite disposés en des emplacements appropriés à l'intérieur de l'automate. Ces machines comprennent donc des moyens de préhension et de transfert automatiques relativement complexes, cause de défaillances, et coûteux. Elles conviennent aux laboratoires ayant des nombres importants de tests à effectuer, en rapport avec les cadences précédemment évoquées et qui possèdent une culture et une compétence en automatismes de laboratoires.

Parallèlement, il existe, à l'usage des petits laboratoires qui ne possèdent pas de compétences particulières en automatismes, des machines manuelles ou semi-automatiques présentant une grande fiabilité (taux d'intervention suite à des défaillances, toute cause confondue, de l'ordre de 1 intervention/10 ans).

Il s'avère qu'entre les deux types d'appareils précédemment évoqués, il existe un marché inoccupé correspondant à des machines automatiques de taille petite et moyenne pouvant fonctionner à des cadences de l'ordre de 60 à 120 tests/heure et devant présenter une grande fiabilité du fait de la structure et de la compétence des laboratoires auxquels ces machines s'adressent.

Par le brevet EP 0 525 273 A1, on a également proposé un dispositif pour la détermination du temps de coagulation du sang faisant intervenir une plaque dans laquelle sont disposées une pluralité de cavités dans lesquelles sont disposées des billes avec les échantillons. Cette plaque est soumise à un mouvement circulaire sous l'effet d'un moteur.

Selon ce document une caméra électronique réalise une image de la plaque avec toutes ses cavités en vue de détecter des variations de déplacement des billes

Il s'avère que la complexité des solutions utilisées pour l'automatisation et ses coûts correspondants, constituent un frein à la diffusion dans les petits et moyens laboratoires. Dans le premier cas, cette complexité est principalement due aux manipulations des cuvettes, nécessaires dans le cycle d'analyse pour effectuer leur préhension, les détacher du film, les amener dans le puits de mesure et pour les extraire en fin de mesure en vue de les rejeter dans un compartiment de décharge, étant entendu que ces manipulations s'avèrent nécessaires en raison de la nature des moyens utilisés pour la détermination du temps de coagulation du sang.

L'invention a donc plus particulièrement pour but la réalisation d'un appareil de conception plus simple et moins coûteuse et qui autorise cependant des cadences suffisantes de manière à pouvoir occuper le domaine des appareils de dimensions petites et moyennes. A cet effet, elle propose un appareil d'analyse automatique du type dans lequel un échantillon liquide à analyser est disposé dans une cuvette contenant une bille ferromagnétique entraînée dans un mouvement périodique sous l'effet d'un champ magnétique, cet appareil comprenant une caméra électronique orientée vers la cuvette et un processeur apte à détecter à partir de l'image de la cuvette les modifications des mouvements de la bille.

Selon l'invention, cet appareil est caractérisé en ce qu'il utilise une pluralité de cuvettes fixées sur une bande de support défilant une à une dans un poste de détection, et en ce que le poste de détection comprend au moins une caméra électronique située au-dessous des cuvettes et des moyens électromagnétiques placés latéralement par rapport à la bande au droit des faces latérales des cuvettes, de manière à engendrer un champ magnétique dans l'axe de déplacement de la bille contenue dans la cuvette se trouvant dans le poste de détection, le mouvement de défilement de la bande pouvant être de type pas à pas ou même continu.

Avantageusement, la caméra électronique sera disposée au-dessous de la cuvette, les moyens électromagnétiques destinés à engendrer le champ magnétique extérieur étant alors disposés latéralement, dans l'axe de déplacement de la bille.

Il s'avère que cette association caméra/moyens électromagnétiques se prête particulièrement bien à la détection en ligne des mouvements des billes contenues dans les cuvettes.

Dans ce cas, les cuvettes, qui demeurent fixées à la bande support souple, sont entraînées selon un mouvement pas à pas, voire même éventuellement continu, le long d'un trajet passant successivement par un poste de pipetage dans lequel s'effectue une injection de produit réactif, un poste de détection comprenant des moyens électromagnétiques placés latéralement, de part et/ou d'autre de la bande, et au moins une caméra électronique disposée sous le trajet des cuvettes dans la zone d'émission du champ magnétique, et un poste de stockage des cuvettes usagées.

Le poste de pipetage pourra avantageusement comprendre une pipette mobile à la fois verticalement et selon un trajet circulaire horizontal de manière à pouvoir occuper une position d'injection située au droit du trajet des cuvettes afin de permettre l'injection de réactifs à l'intérieur de celles-ci, une position de rinçage située au droit d'une cuve de rinçage et au moins une position de prélèvement de réactif située au droit d'une aire de prélèvement d'un distributeur de réactif.

Ce distributeur de réactif pourra consister en un carrousel comprenant une pluralité de cuves de réactif mobiles selon un trajet circulaire de manière à pouvoir être amenées successivement dans l'aire de prélèvement.

Un mode d'exécution de l'invention sera décrit ci-après avec référence aux dessins annexés dans lesquels :

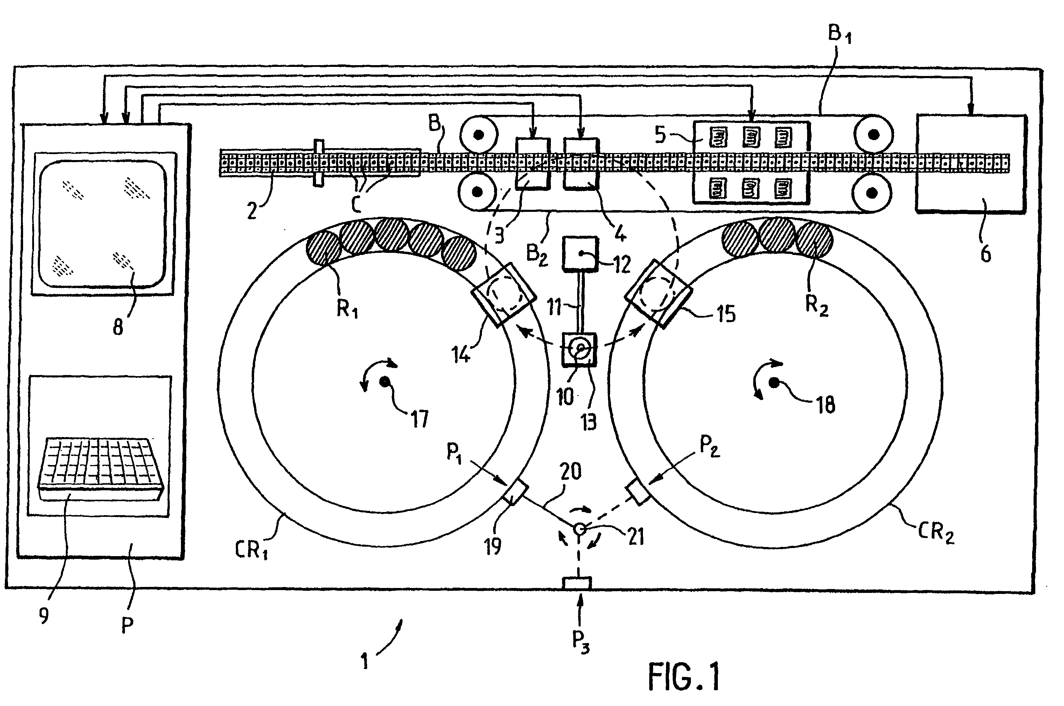

La figure 1 est une représentation schématique d'un appareil d'analyse automatique de dimension moyenne ;
La figure 2 est une vue en perspective schématique d'une cuvette montée sur sa bande ;
La figure 3 est une vue schématique de dessus de la bande équipée de ses cuvettes lors de son passage par les postes d'incision, de pipetage et de détection ;
La figure 4 est une coupe schématique verticale selon A/A de la figure 3;
La figure 5 est une vue schématisant les déplacements de la bille contenue dans une cuvette, à l'intérieur du champ d'une caméra ;
La figure 6 est un graphique montrant l'amplitude des mouvements de la bille en fonction du temps.

Dans cet exemple, l'appareil d'analyse automatique 1 fait intervenir une alimentation en cuvettes comportant une bobine 2 sur laquelle est enroulée une bande B portant une pluralité de cuvettes C.

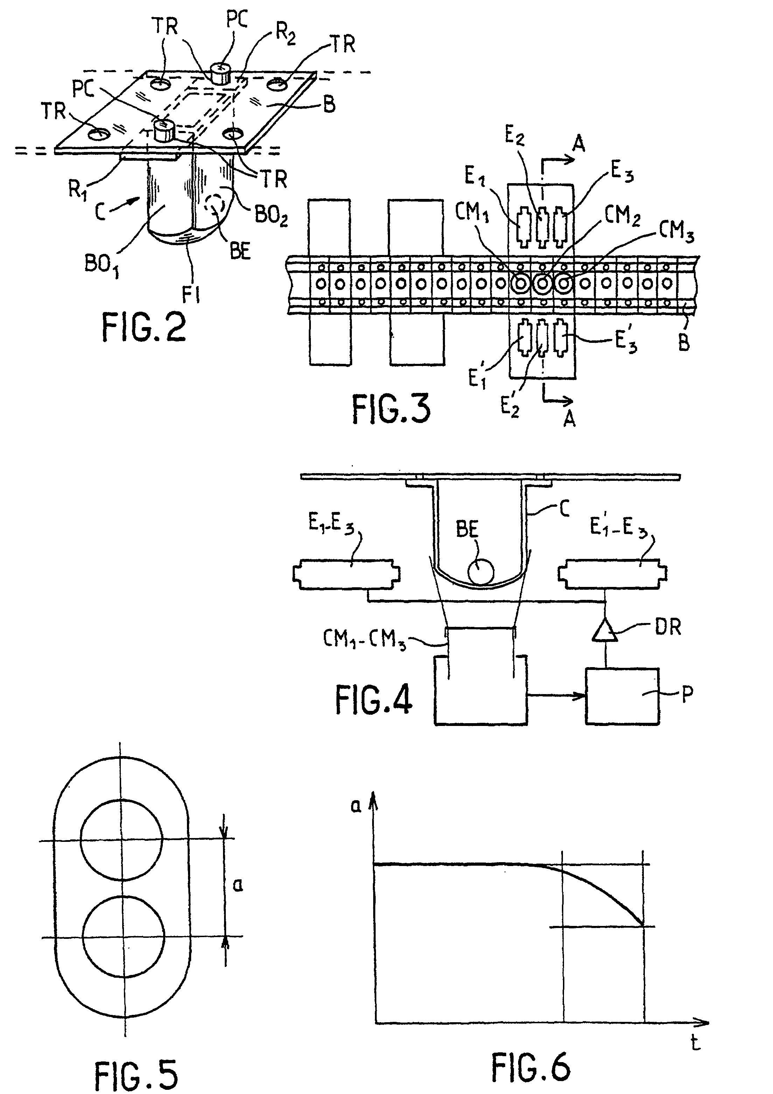

Comme illustré sur la figure 2, les cuvettes C réalisées par moulage d'une matière plastique transparente, présentent chacune un corps de forme plate parallélépipédique dont le fond incurvé FI constitue un chemin de roulement pour une bille BE en matériau ferromagnétique. A l'opposé de ce fond FI, la cuvette C présente une ouverture au niveau de laquelle ses deux bords opposés BO₁, BO₂ sont prolongés à angle droit par deux rebords respectifs R₁, R₂ munis chacun d'une protubérance cylindrique PC s'étendant du côté opposé au corps. Ces deux protubérances sont destinées à venir s'engager à force dans deux trous respectifs TR respectivement prévus sur les deux bordures latérales de la bande B.

La bobine 2 est montée rotative à l'intérieur d'un réceptacle de manière à permettre un déroulement de la bande B selon un trajet rectiligne passant successivement par un poste d'incision 3 de la bande B (dans le cas où la bande n'est pas déjà préincisée), un poste de pipetage 4, un poste de détection 5, puis un poste de récupération 6 de la bande B munie des cuvettes usagées.

Le fonctionnement de ces différents postes est géré par un processeur P comportant une unité centrale ainsi que des périphériques tels que, par exemple, un ensemble écran 8/clavier 9.

L'entraînement de la bande B le long de son trajet est assuré au moyen d'un mécanisme d'entraînement pas à pas faisant intervenir deux bandes sans fin B₁, B₂ guidées par des galets et prenant appui sur les faces latérales des cuvettes C portées par la bande B.

Le poste de pipetage 4 est desservi par une pipette verticale automatisée 10, mobile en hauteur, de manière à pouvoir prendre une position basse de pipetage ou de rinçage et une position haute permettant ses déplacements dans un plan horizontal.

Cette pipette 10 est fixée à l'une des extrémités d'un bras 11 monté rotatif, par son autre extrémité, autour d'un axe vertical 12. L'entraînement en rotation du bras 11 est assuré par un servomoteur commandé par le processeur P.

Grâce à ce mécanisme particulièrement simple, la pipette 10 peut être amenée successivement à l'aire de pipetage du poste de pipetage 4, à une aire de rinçage 13 diamétralement opposée, équipée d'une ou plusieurs cuves de rinçage et à deux aires de prélèvement 14, 15 disposées symétriquement par rapport à l'axe passant par l'aire de pipetage 4 et l'aire de rinçage 13.

Les aires de prélèvement 14, 15 sont situées dans le trajet de récipients R₁, R₂ portés par deux carrousels respectifs CR₁, CR₂ mobiles en rotation autour de deux axes verticaux 17, 18 et commandés par deux servomoteurs pilotés par le processeur P.

L'un de ces carrousels CR₁ est destiné à contenir les récipients R₁ d'échantillons sanguins à analyser tandis que l'autre CR₂ contient des récipients R₂ affectés aux différents réactifs utilisables dans le cadre des analyses que l'on veut effectuer.

Bien entendu, le processeur P est programmé de manière à commander des séquences de pipetage appropriées à la nature des analyses à effectuer, et pouvant comprendre successivement :
- un rinçage préalable de la pipette 10,
- le prélèvement d'une dose d'échantillons contenu dans l'un des récipients R₁ du carrousel CR₁,
- l'injection de cette dose dans une cuvette C située dans le poste de pipetage 4,
- le rinçage de la pipette 10,
- le prélèvement d'une dose de réactif contenu dans l'un des récipients R₂ du carrousel CR₂,
- l'injection de cette dose de réactif dans la cuvette C, l'identification des échantillons sanguins à analyser ainsi que celle des réactifs s'effectue automatiquement grâce à un lecteur de code à barres 19 apte à effectuer la lecture de codes à barres présents sur les récipients R₁, R₂ portés par les carrousels CR₁, CR₂.

Dans cet exemple, on utilise pour ces lectures un lecteur de codes à barres unique 19 monté à l'extrémité d'un bras 20 pivotant autour d'un axe vertical 21 de manière à pouvoir occuper trois positions, à savoir :
- une position P₁ de lecture des codes à barres des récipients R₁ du carrousel CR₁,
- une position P₂ de lecture des codes à barres des récipients R₂ du carrousel CR₂, et
- une position P₃ de lecture de récipients placés par l'opérateur dans un poste de lecture, par exemple en vue de la saisie des informations exploitées par le processeur dans le cadre du fonctionnement de l'appareil.

Le poste de mesure 4 comprend ici trois unités de mesures successives comprenant chacune (figures 3 et 4) :
- un couple d'électroaimants coaxiaux E₁, E'₁ - E₂, E'₂ - E₃, E'₃ situés de part et d'autre de la bande B, au droit des faces latérales des cuvettes C, et
- une caméra électronique CM₁, CM₃ dont l'objectif est situé au-dessous des cuvettes C portées par la bande B.

Les électroaimants E₁, E'₁ - E₂, E'₂- E₃, E'₃ sont excités par un circuit de puissance PR piloté par le processeur P de manière à engendrer un champ magnétique impulsionnel susceptible d'entraîner la bille BE selon un déplacement alternatif au fond de la cuvette C.

La caméra CM₁ - CM₃ est couplée au processeur P qui analyse, en temps réel, l'image grâce à un logiciel approprié, de manière à mesurer l'amplitude a des oscillations de la bille BE et déterminer l'instant critique où cette amplitude s'abaisse en dessous d'un seuil déterminé (par exemple 50 % de l'amplitude initiale) (figures 5 et 6).

Bien entendu, le processeur P effectue un comptage du temps entre le moment où le réactif a été injecté dans la cuvette C et cet instant critique, de manière à en déduire un temps de coagulation.

L'emploi de plusieurs unités de mesure échelonnées sur le trajet de la bande B présente l'avantage d'autoriser une plus grande souplesse de fonctionnement et surtout d'étendre considérablement la plage des temps de coagulation des échantillons sanguins à analyser.

Bien entendu, les déplacements pas à pas de la bande sont synchronisés avec les temps de fonctionnement de chacun des postes de l'appareil et notamment avec les impulsions de champ magnétique engendrées par les bobines.

Eventuellement, le poste de pipetage pourra être situé en un même emplacement que le poste de mesure.

Bien entendu, l'invention ne se limite pas au mode d'exécution précédemment décrit.

Ainsi, par exemple, chaque caméra pourra présenter un champ incluant plusieurs cuvettes excitées chacune par un couple d'électroaimants distinct, de manière à suivre la cuvette sur une avance de plusieurs pas avec un processeur P programmé de manière à détecter simultanément les déplacements des billes de différentes cuvettes.

## Revendications

1. Appareil d'analyse automatique du type dans lequel un échantillon liquide à analyser est disposé dans une cuvette (C) contenant une bille ferromagnétique (BE) entraînée dans un mouvement périodique sous l'effet d'un champ magnétique, cet appareil comprenant une caméra électronique (CM₁ à CM₃) orientée vers la cuvette (C) et un processeur (P) apte à détecter à partir de l'image de la cuvette (C) les modifications des mouvements de la bille (BE), **caractérisé en ce qu'**il utilise une pluralité de cuvettes fixées sur une bande de support (B) défilant une à une dans un poste de détection (5), et **en ce que** le poste de détection comprend au moins une caméra électronique située (CM₁, CM₂) au-dessous des cuvettes et des moyens électromagnétiques placés latéralement par rapport à la bande (B) au droit des faces latérales des cuvettes, de manière à engendrer un champ magnétique dans l'axe de déplacement de la bille contenue dans la cuvette se trouvant dans le poste de détection, le mouvement de défilement de la bande (B) pouvant être de type pas à pas ou même continu.

2. Appareil selon la revendication 1,
**caractérisé en ce que** la bande (B) suit un trajet rectiligne passant successivement par un poste d'incision de la bande (B) éventuel, un poste de pipetage (4), un poste de détection (5) et un poste de récunération (6) de la bande munie de ses cuvettes usagées.

3. Appareil selon l'une des revendications précédéntes,
**caractérisé en ce que** le poste de pipetage (4) est desservi par une pipette verticale (10) mobile en hauteur fixée à l'une des extrémités d'un bras (11) monté rotatif autour d'un axe vertical de manière à pouvoir être amené par rotation successivement, à l'aire de pipetage, à une aire de rincage et à deux aires de prélèvement (14, 15).

4. Appareil selon la revendication 3,
**caractérisé en ce que** les aires de prélèvement (14, 15) sont situées dans le trajet de récipients (R₁, R₂) portés par deux carrousels respectifs (CR₁, CR₂) mobiles en rotation autour de deux axes verticaux (17, 18) commandés par deux servomoteurs, l'un des carrousels (CR₁) étant destiné à contenir les récipients (R₁) d'échantillons sanguins, tandis que l'autre (CR₂) contient des récipients (R₂) affectés aux réactifs utilisables dans le cadre des analyses que l'on veut effectuer.

5. Appareil selon la revendication 4,
**caractérisé en ce qu'**il comprend un processeur (P) programmé de manière à commander des séquences de pipetage comportant successivement :
- un rinçage préalable de la pipette (10),
- le prélèvement d'une dose d'échantillons contenu dans l'un des récipients (R₁) du carrousel (CR₁),
- l'injection de cette dose dans une cuvette (C) située dans le poste de pipetage (4),
- le rinçage de la pipette (10),
- le prélèvement d'une dose de réactif contenu dans l'un des récipients (R₂) du carrousel (CR₂),
- l'injection de cette dose de réactif dans la cuvette (C).

6. Appareil selon la revendication 5,
- **caractérisé en ce que** l'identification des échantillons sanguins à analyser ainsi que celle des réactifs s'effectue automatiquement grâce à un lecteur de code à barres (19) apte à effectuer la lecture de codes à barres présents sur les récipients (R₁, R₂) portés par les carrousels (CR₁, CR₂).

7. Appareil selon la revendication 6,
**caractérisé en ce qu'**il comprend un lecteur de codes à barres unique (19) monté à l'extrémité d'un bras (20) pivotant autour d'un axe vertical (21) de manière à pouvoir occuper trois positions, à savoir :
- une position (P₁) de lecture des codes à barres des récipients (R₁) du carrousel (CR₁),
- une position (P₂) de lecture des codes à barres des récipients (R₂) du carrousel (CR₂), et
- une position (P₃) de lecture de récipients placés par l'opérateur dans un poste de lecture, par exemple en vue de la saisie des informations exploitées par le processeur dans le cadre du fonctionnement de l'appareil.

8. Appareil selon l'une des revendications précédentes,
**caractérisé en ce que** le champ de la caméra (CM₁ - CM₃) inclut plusieurs cuvettes (C) excitées chacune par des moyens électromagnétiques distincts.

9. Appareil selon la revendication 8,
**caractérisé en ce que** le processeur (P) est programmé de manière à détecter simultanément les déplacements des billes (BE) desdites cuvettes (C).

## Patentansprüche

1. Gerät für die automatische Analyse, von dem Typ, bei dem eine zu analysierende flüssige Probe in einen Behälter (C) gebracht wird, der eine ferromagnetische Kugel (BE) enthält, die unter Einwirkung eines Magnetfeldes in eine regelmäßige Bewegung versetzt wird, wobei das Gerät eine elektronische Kamera (CM₁, CM₃) beinhaltet, die auf den Behälter (C) gerichtet ist, und einen Prozessor (P), der in der Lage ist, anhand des Bildes des Behälters (C) Veränderungen der Bewegungen der Kugel (BE) festzustellen, **dadurch gekennzeichnet, dass** das Gerät eine Vielzahl von Behältern verwendet, die an einem Transportband (B) befestigt sind und nacheinander an einer Sensorstation (5) vorbeilaufen, und dadurch, dass die Sensorstation mindestens eine elektronische Kamera (CM₁, CM₃) beinhaltet, die unter den Behältern angeordnet ist, und elektromagnetische Vorrichtungen, die im Verhältnis zum Band (B) seitlich und gegenüber den Seitenflächen der Behälter angeordnet sind, so dass in der Bewegungsachse der Kugel, die sich in dem Behälter befindet, der sich gerade in der Sensorstation befindet, ein Magnetfeld erzeugt wird, wobei die Bewegungsart des Bandes (B) eine schrittweise oder auch eine gleichmäßige Bewegung sein kann.

2. Gerät gemäß Patentanspruch 1,
**dadurch gekennzeichnet, dass** das Band (B) einem geradlinigen Weg folgt, der nacheinander an einer eventuellen Station für das Einschneiden des Bandes (B), einer Pipettenstation (4), einer Sensorstation (5) und einer Auffangstation (6) für das Band mit den gebrauchten Behältern vorbeiführt.

3. Gerät gemäß einem der voranstehenden Patentansprüche,
**dadurch gekennzeichnet, dass** die Pipettenstation (4) von einer senkrecht angeordneten Pipette (10) bedient wird, deren Höhe verstellbar ist und die an einem Ende eines Amis (11) befestigt ist, der um eine senkrechte Achse drehbar montiert ist, so dass er durch Rotation nacheinander in den Pipettierbereich, in einen Spülbereich und in zwei Entnahmebereiche (14, 15) geführt werden kann.

4. Gerät gemäß Patentanspruch 3,
**dadurch gekennzeichnet, dass** die Entnahmebereiche (14, 15) auf dem Arbeitsweg der Behälter ( R₁, R₂) angeordnet sind, die von zwei Karussells (CR₁, CR₂) getragen werden, die sich um zwei senkrechte Achsen (17, 18) drehen, die von zwei Servomotoren betätigt werden, wobei eines der Karussells (CR₁) für die Aufnahme der Behälter (R₁) mit den Blutproben bestimmt ist, während das andere (CR₂) Behälter (R₂) für die Reaktionsmittel enthält, die im Rahmen der durchzuführenden Analysen verwendet werden können.

5. Gerät gemäß Patentanspruch 4,
**dadurch gekennzeichnet, dass** es einen Prozessor (P) beinhaltet, der so programmiert ist, dass er Pipettiersequenzen steuert, die nacheinander folgende Schritte beinhalten:
- einen vorausgehenden Spülvorgang der Pipette (10),
- die Entnahme einer Probendosis, die in einem der Behälter (R₁) von Karussell (CR₁) enthalten sind,
- die Injektion dieser Dosis in einen Behälter (C), der sich in der Pipettenstation (4) befindet,
- das Spülen der Pipette (10),
- die Entnahme einer Dosis eines Reaktionsmittels, das in einem der Behälter (R₂) von Karussell (CR₂) enthalten ist,
- Injektion dieser Dosis eines Reaktionsmittels in den Behälter (C).

6. Gerät gemäß Patentanspruch 5,
**dadurch gekennzeichnet, dass** die Identifizierung der zu analysierenden Blutproben sowie der Reaktionsmittel automatisch mithilfe eines Strichcodelesers (19) erfolgt, der in der Lage ist, die Strichcodes zu lesen, die auf den Behältern ( R₁, R₂) angebracht sind sind, die in den Karussells (CR₁, CR₂) stehen.

7. Gerät gemäß Patentanspruch 6,
**dadurch gekennzeichnet, dass** es einen einzigen Strichcodeleser (19) beinhaltet, der am Ende eines Arms (20) montiert ist, der sich so um eine senkrechte Achse (21) dreht, dass er drei Positionen einnehmen kann, nämlich
- eine Position (P₁) für das Lesen der Strichcodes auf den Behältern (R₁) von Karussell (CR₁),
- eine Position (P₂) für das Lesen der Strichcodes auf den Behältern (R₂) von Karussell (CR₂),
- eine Position (P₃) für das Lesen der Behälter, die vom Bediener in eine Lesestation gestellt werden, z.B. um Informationen zu erfassen, die vom Prozessor im Rahmen des Gerätebetriebs verwendet werden.

8. Gerät gemäß irgendeinem der voranstehenden Patentansprüche,
**dadurch gekennzeichnet, dass** das Blickfeld der Kamera (CM₁ - CM₃) mehrere Behälter C) einschließt, die jeweils von verschiedenen elektromagnetischen Vorrichtungen erregt werden,

9. Gerät gemäß Patentanspruch 8,
**dadurch gekennzeichnet, dass** der Prozessor (P) so programmiert ist, dass er simultan die Bewegungen der Kugeln (BE) in den genannten Behältern (C) feststellt.

## Claims

1. Automatic analysis apparatus of the type in which a liquid sample to be analysed is placed in a cup (C) containing a ferromagnetic ball (BE) driven in a periodic movement under the effect of a magnetic field, this apparatus including an electronic camera (CM₁ to CM₃) orientated towards the cup (C), and a processor (P) for detecting from the image of the cup (C) the modifications of the movements of the ball (BE), **characterised in that** it uses a plurality of cups secured to a support strip (B) running one by one into a detection station (5), and **in that** the detection station includes at least one electronic camera (CM₁, CM₂) situated below the cups and electromagnetic means placed laterally with respect to the strip (B) at the right of the side faces of the cups so as to generate a magnetic field inside the movement axis of the ball contained in the cup located in the detection station, the unwinding movement of the strip (B) able to be of the step-by-step or even continuous type.

2. Apparatus according to claim 1, **characterised in that** the strip (B) follows a rectilinear path passing successively through a possible incision station of the strip (B), a pipette station (4), a detection station (5) and a recovery station (6) of the strip provided with its used cups.

3. Apparatus according to one of the preceding claims, **characterised in that** the pipette station (4) is served by a vertical pipette (10) abler to move upwards and fixed to one of the extremities of an arm (11) mounted rotating around a vertical spindle so as to be able to be brought by successively rotating to a pipette station, a rinsing station and to two sampling areas (14, 15).

4. Apparatus according to claim 3, **characterised in that** the sampling areas (14, 15) are situated on the path of the receptacles (R₁, R₂) borne by two respective carrousels (CR₁, CR₂) able to move in rotation around two vertical spindles (17, 18) controlled by two servomotors, one of the carrousels (CR₁) being used to contain the blood samples receptacles (R₁), whereas the other (CR₂) contains the receptacles (R₂) allocated to the reactive agents able to be used as part of the analyses it is desired to be carried out.

5. Apparatus according to claim 4, **characterised in that** it includes a processor (P) programmed so as to control pipette sequences successively comprising:
- a prior rinsing of the pipette (10),
- the taking of a dose of samples contained in one of the receptacles (R₁) of the carrousel (CR₁),
- the injection of this dose into a cup (C) situated on the pipette station (4),
- the rinsing of the pipette (10),
- the taking of a dose of the reactive agent contained in one of the receptacles (R₂) of the carrousel (CR₂),
- the injection of this reactive agent dose into the cup (C).

6. Apparatus according to claim 5, **characterised in that** the blood samples to be analysed and that of the reactive agents is made automatically by means of a bar code reader (19) able to read the bar codes present on the receptacles (R₁, R₂) borne by the carrousels (CR₁, CR₂).

7. Apparatus according to claim 6, **characterised in that** it includes a single bar code reader (19) mounted at the extremity of an arm (20) pivoting around a vertical spindle (21) so as to be able to occupy three positions, namely:
- a position (P₁) for reading the bar codes of the receptacles (R₁) of the carrousel (CR₁),
- a position (P₂) for reading bar codes of the receptacles (R₂) of the carrousel (CR₂), and
- a position (P₃) for reading receptacles placed by the operator in a reading station, for example for picking up information exploited by the processor as part of the functioning of the apparatus.

8. Apparatus according to one of the preceding claims, **characterised in that** the field of the camera (CM₁ - CM₃) includes several cups (C) each excited by separate electromagnetic means.

9. Apparatus according to claim 8, **characterised in that** the processor (P) is programmed so as to simultaneously detect the movements of the balls (BE) of said cups (C).
